(19) Europäisches Patentamt
European Patent Office
Office européen des brevets

(11) Publication number: **0 194 144 B1**

# (12) EUROPEAN PATENT SPECIFICATION

(45) Date of publication of patent specification: **11.09.91**

(51) Int. Cl.5: **C07C 45/59**, C07C 49/597, C07C 221/00, C07C 255/31, C07C 69/738, //C07D307/32, C07D303/42

(21) Application number: **86301568.1**

(22) Date of filing: **05.03.86**

The file contains technical information submitted after the application was filed and not included in this specification

## (54) Process for the preparation of 2-Alkyl-Cyclopent-2-Enones.

(30) Priority: **05.03.85 IT 1977085**

(43) Date of publication of application: **10.09.86 Bulletin 86/37**

(45) Publication of the grant of the patent: **11.09.91 Bulletin 91/37**

(84) Designated Contracting States: **BE CH DE FR GB LI NL SE**

(56) References cited:
**FR-A- 765 515**

**LA FRANCE ET SES PARFUMS, no. 3, 1958, pages 39-53; M.M. BORNAND et al.: "Les synthèses de la jasmone et de ses dérivès"**

**COMPTES RENDUS ACAD. SC., no. 234, 1952, pages 1694-1696; R. ROTHSTEIN et al.: "Sur l'ouverture des oxydes d'éthylène par l'ester malonique sodé. Synthèse de la dihydrojasmone"**

(73) Proprietor: **Montedison S.p.A.**
**31, Foro Buonaparte**
**I-20121 Milan(IT)**

(72) Inventor: **Dalcanale, Enrico**
**14, Via Visentin**
**I-28100 Novara(IT)**

(74) Representative: **Whalley, Kevin et al**
**MARKS & CLERK 57/60 Lincoln's Inn Fields**
**London WC2A 3LS(GB)**

**EP 0 194 144 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

COMPTES RENDUS ACAD. SC., no. 234, 17th March 1952, pages 1293-1295; R. ROTHSTEIN et al.: "Sur l'ouverture du cycle des oxydes d'ethylène par l'ester malonique sodé"

L.F. FIESER et al.: "Reagents for Organic Synthesis", 1967, vol. I, pages 136,137, John Wiley and Sons, Inc.

J. ORG. CHEM., vol. 48, no. 21, 1983, pages 3831-3833, American Chemical Society, Washington, D.C., US; C. VENTURELLO et al.: "A new, effective catalytic system for epoxidation of olefins by hydrogen peroxide under phase-transfer conditions"

J. ORG. CHEM., vol. 29, September 1964, page 2810; C.H. DePUY et al.: "The reaction of diethyl malonate with styrene oxide"

J. ORG. CHEM., vol. 38, no. 23, 1973, pages 4071-4073; P.E. EATON et al.: "Phosphorus pentoxide-methanesulfonic acid. A convenient alternative to polyphosphoric acid"

EXPERIENTIA, vol. XI/3, 1955, pages 114-115, Birkhäuser Verlag, Basel, CH; CH. RAI et al.: "Cyclopentenones from Lactones"

R. ROTHSTEIN, Bull. Soc. Chim., 5e serie, 1935, p. 1936

## Description

The present invention relates to a process for the preparation of 2-alkyl-cyclopent-2-enones of formula

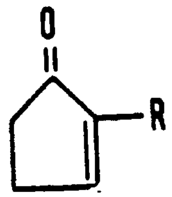

(I)

wherein R represents one of the following groups: $C_nH_{2n+1}$ (the chain being linear or branched);
$(CH_2)_n$-COOR';
$(CH_2)_n$-NR'$_2$;
$(CH_2)_n$-OR';
$(CH_2)_nX$ wherein X = Cl, Br, I or F;
$(CH_2)_n$-CN.
In each of these groups, n is from 1 to 20 and R' is an alkyl radical containing up to 5 carbon atoms, a benzyl radical or a phenyl radical, the benzyl or phenyl radicals being optionally substituted in the aromatic nucleus by one or more groups inert under the reaction conditions.

Cyclopentenones (I) are useful intermediates for the manufacture of pharmaceutical products and of drugs for veterinary use, in particular prostaglandin.

According to a known process for preparing cyclopentenones, an olefin is reacted, in a first step. with trihydrated manganese acetate, potassium permanganate, acetic anhydride and anhydrous sodium acetate, all in a large excess, with respect to the olefin, thereby obtaining a γ-alkyl lactone, which, in a second step, is cyclized by means of polyphosphoric acid, thereby obtaining the final product. Such a process gives rise to low yields and requires particular reactants, which are expensive and may be handled only with difficulty; furthermore the reactions lead to complex mixtures of products from which the γ-alkyl lactone and the final product may be separated only with difficulty.

In the disclosure La France et ses Parfums, 1958, no. 3, pages 39-53, there is described (at page 51, paragraph 2) a process for the preparation of 2-alkyl-3-methyl-cyclopent-2-enones by the following steps:

1)an α-epoxide is reacted with an alkylating agent of formula Na-CH(COOEt)$_2$ to obtain an α-carbalkoxy-γ-alkyl lactone,

2)the α-carbalkoxy-γ-alkyl lactone is treated to obtain a γ-alkyl lactone,

3)the γ-alkyl-lactone is reacted to obtain by cyclization the 2-alkyl-3-methyl-cyclopent-2-enone.

The invention aims to provide a process for the preparation of 2-alkyl-cyclopent-2-enones of general formula (I) with reasonably good yields, which process starts from raw materials which are cheap and can be handled easily, and which process allows the intermediate products and the final product to be separated easily.

The invention accordingly provides a process for the preparation of 2-alkyl-cyclopent-2-enones of formula (I), characterized by comprising the following steps:

1) an α-olefin of formula $CH_2$=CH-$CH_2$-R (II) is oxidized to an epoxide of formula (III)

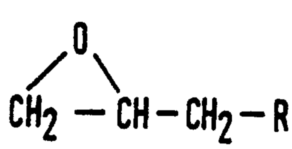

(III)

2) the epoxide (III) is reacted under reflux with an alkylating agent of formula $Na^+[CH(COOR'')_2]^-$ wherein R'' is an ethyl, isopropyl or isobutyl radical, in the presence of a malonic ester of formula COOR''-$CH_2$-COOR'', in an alcoholic solvent of formula R''OH, the alkylating agent being used in a substantially equimolar ratio, with respect to the epoxide, and wherein said malonic ester is used in a molar ratio, with respect to the epoxide, of from 0.5 to 2, to obtain an α-carbalkoxy-γ-alkyl lactone of formula (IV)

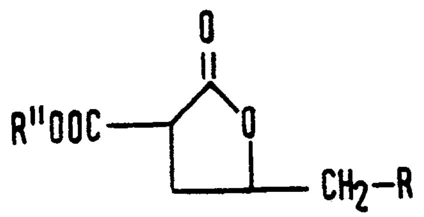

(IV)

3) the α-carbalkoxy-γ-alkyl lactone (IV) is saponified in an alkaline medium, to thereby obtain the corresponding acid;

4) the corresponding acid is decarboxylated, at 100 - 160 °C, to thereby obtain a γ-alkyl lactone of formula (V)

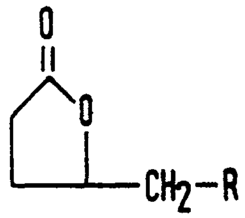

(V)

5) the γ-alkyl lactone (V) is reacted at 20 - 100 °C with a protic acid, to thereby obtain, by cyclization, a 2-alkyl-cyclopent-2-enone of formula (I).

In the starting olefin (and consequently in the 2-alkyl-cyclopent-2-enone (I) as well), n is preferably from 1 to 10, and R is preferably $C_nH_{2n+1}$ or $(CH_2)_n$-COOR'. When radical R' is a benzyl or a phenyl radical, these groups may be substituted in the aromatic nucleus by one or more (usually 1 or 2) inert groups preferably selected from $NR_2$''', OR''', COOR''', X, CN, $NO_2$, where X has the meaning hereinbefore and where R''' is an alkyl radical containing up to 5 carbon atoms, a benzyl radical or a phenyl radical.

The oxidization of olefin $CH_2=CH-CH_2-R$ (II) to epoxide (III) can be carried out by means of well-known methods, according to which the substituents, optionally present in group R, remain unaltered. A suitable method resides in oxidizing the olefin by means of m-chloroperbenzoic acid; such method provides the epoxide with yields over 90% and allows a very simple working. Another suitable method resides in oxidising the olefin with $H_2O_2$ in the presence of a catalyst based on tungstate and phosphate ions; such method is described for instance in an article of C. Venturello et al, in J. Org. Chem. 48, 3831, 1983. This last method is more economic than the first one, gives rise to good epoxide yields (about 70%) and allows recovery of a lot of non-reacted olefin. Nevertheless still other methods may be used, provided they do not alter the substituents, optionally present in group R.

The second step of the process according to the invention resides in alkylating the epoxide (III) by means of an alkylating agent $Na^+[CH(COOR'')_2]^-$, where R'' is an ethyl, isopropyl or isobutyl radical; the reaction is described for instance in the article of C. H. Depuy et al, in J. Org. Chem., 1964, page 2810, the reaction scheme being:

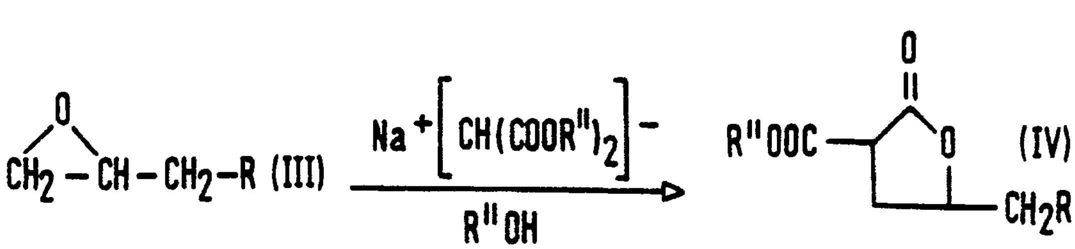


(IV) (1)

The epoxide is reacted with the above mentioned alkylating agent in the presence of a malonic ester of formula COOR''-$CH_2$-COOR''. The alkylating agent should be used in a substantially equimolar ratio, with respect to the epoxide, and said malonic ester should be used in a molar ratio, with respect to the epoxide, of from 0.5 to 2, preferably about 1. The reaction has to be carried out under reflux conditions. The alkylation of epoxide (III) is found to be completely regioselective, providing only the attack product in the

non-substituted position.

An intramolecular cyclization, yielding lactone (IV), follows the alkylation. The reaction can be carried out suitably as follows: metal sodium, in a finely subdivided form, is added to an anhydrous alcohol R"OH. When the whole of the sodium is dissolved, the malonic ester COOR"-$CH_2$-COOR" is added in such an amount as to obtain in solution both the alkylating agent and the non-reacted malonic ester, according to the molar ratios defined hereinbefore. The alkylating agent $Na^+[CH(COOR")_2]^-$ forms immediately. At this point the solution is brought under reflux conditions and the epoxide is added gradually; the whole is made to react for a time of from 1 to 8 hours, depending on substituent R.

α-carboxy-γ-alkyl lactone (IV) is then saponified in an alkaline medium, thereby obtaining the corresponding acid (VI).

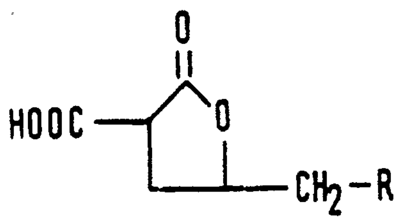

(VI)

The reaction can be carried out as follows: an aqueous solution of sodium carbonate is added to the reaction mixture of the preceding step and the two phases are mixed generally at from 50 °C to the reflux temperature.

Acid (VI) can be separated from the reaction mixture as follows: alcohol R"OH is distilled, while leaving the acid in the aqueous phase and separating said acid successively by crystallization or by extraction by means of an organic solvent.

Acid (VI) is then decarboxylated, at 100° - 160 °C, thereby obtaining a γ-alkyl lactone of formula (V). The decarboxylation is preferably carried out at 120° - 140 °C. Some of the γ-alkyl lactones can be used for perfumes or essential oils.

The γ-alkyl lactone (V) is then reacted at 20 - 100 °C (preferably 50-60 °C), with a protic acid, thereby obtaining, by cyclization, a 2-alkyl-cyclopent-2-enone of formula (I).

The protic acids are generally selected from polyphosphoric acid, 96% sulphuric acid (weight/volume), and a mixture consisting of $CH_3$-$SO_3H$ and $P_2O_5$. The mixture of $CH_3$-$SO_3H$ and $P_2O_5$ is found to be particularly suitable. In such mixture the $CH_3$-$SO_3H/P_2O_5$ weight ratio is usually from 10 to 20 and is more preferably equal to about 10. The preparation of the mixture of $CH_3$-$SO_3H$ and $P_2O_5$ is described for instance in an article of P. E. Eaton et al, in J. Org. Chem. 38, 4071, 1973, and the cyclization reaction by means of a protic acid is described for instance in an article of E. Uhlig et al, in Adv. Org. Chem. 1, 35, 1960.

The reaction can be carried out as follows: γ-alkyl lactone (V) is added to the protic acid, at room temperature. The resultant solution is heated as defined hereinbefore for a time generally from 5 to 24 hours, depending on the nature of substituent R. Then the solution is poured into water and the resultant solution is extracted by means of an organic solvent immiscible with water. The organic phase is separated, dehydrated and concentrated, in order to yield an oil. The product is then isolated by distillation under reduced pressure, by crystallization or by separation on a chromatographic column or by the combined use of two of these techniques.

The main advantages of the process of the present invention may be summarized as follows:

- the operative conditions are very simple;
- the starting materials and the reactants are cheap;
- the first four steps give rise to high yields, and therefore the overall yield of the process is good;
- the intermediate products and the final product can be easily separated.

The invention will be further described with reference to the following illustrative Examples.

Example 1

Step (1a): Epoxidation by means of meta-chloroperbenzoic acid.

53.08 g (0.25 moles) of ethyl-10-undecenoate were introduced into a 1000 $cm^3$ flask, provided with a stirrer, reflux cooler, thermometer, and dropping funnel; then 60.92 g (0.3 moles) of meta-chloroperbenzoic acid (85% concentration), dissolved in 650 $cm^3$ of chloroform, were added dropwise over 2 hours. Once the

addition was completed the mixture was kept under reflux conditions for 2 more hours and then the solution was cooled and shaken with a 10% by weight aqueous solution of sodium bicarbonate. The organic phase was washed with water until neutrality, dehydrated on anhydrous sodium sulphate ($Na_2SO_4$) and dried. The thus obtained oil was distilled under reduced pressure (108-110 °C/2.10$^{-1}$ mm Hg), thereby providing 52.5 g (0.23 moles) of ethyl-10,11-epoxyundecanoate with a yield of 92%.

Step (1b): Epoxidation with hydrogen peroxide.
0.360 g (1.08.10$^{-3}$ moles) of sodium tungstate ($Na_2WO_4.2H_2O$) dissolved in 6 cm$^3$ of water, 0.55 cm$^3$ (2.24.10$^{-3}$ moles) of 40% phosphoric acid (weight/volume) and 20.5 cm$^3$ (2.11.10$^{-3}$ moles) of 35% hydrogen peroxide (weight/volume) were added, in that order, to a 250 cm$^3$ flask, equipped with a mechanical stirrer, reflux cooler and thermometer. The pH was brought to 1.6 by means of an aqueous solution of 30% sulphuric acid at 30% weight/volume. At this point 53.08 g (2.5.10$^{-1}$ moles) of ethyl-10-undecenoate in 10 cc of 1,2-dichloroethane and 0.179 g (4.48.10$^{-4}$ moles) of tricaprylmethylammonium chloride were added. The resultant two-phase mixture was heated under strong stirring at 70 °C for 4 hours, until the whole of the hydrogen peroxide was consumed, and then the mixture was treated with a saturated aqueous solution of $Na_2SO_3$ (in order to eliminate possible traces of $H_2O_2$) and $NaHCO_3$. The organic phase was separated, dehydrated on anhydrous sodium sulphate, concentrated and distilled under reduced pressure.
Two fractions were obtained:

- at 96-98 °C/2.10$^{-1}$ mm Hg: 9.15 g (4.31.10$^{-2}$ moles) of ethyl-10-undecenoate;
- at 108-110 °C/2.10$^{-1}$ mm Hg: 39.4 g (1.73.10$^{-1}$ moles) of ethyl-10,11-epoxyundecanoate. Yield: 69%. Selectivity 83%.

Steps (2), (3) and (4): Alkylation, saponification and decarboxylation.
100 cm$^3$ of anhydrous ethyl alcohol and 1.01 g (4.38.10$^{-2}$ moles) of finely subdivided sodium were added, in that order, to a 500 cm$^3$ flask, provided with a magnetic stirrer, reflux cooler, thermometer and dropping funnel. The mixture was kept at room temperature until the whole of the sodium was dissolved, and then 14.03 g (8.76.10$^{-2}$ moles) of diethylmalonate were added. The whole was kept under reflux conditions and 10.0 g (4.32.10$^{-2}$ moles) of ethyl-10,11-epoxyundecanoate were added, dropwise, over 1 hour. Once the addition was over, the mixture was kept under stirring for about 3 hours.

Then 3.00 g of sodium carbonate in 100 cm$^3$ of water were added and the alcohol was azeotropically distilled. The residual aqueous solution was acidified by means of an aqueous solution of 10% hydrochloric acid (weight/volume) and cooled down. Overnight there was noted the precipitation of 12 g of a crystalline white solid, which was separated and decarboxylated in a nitrogen stream at 140 °C, thereby providing 9.66 g (3.98.10$^{-2}$ moles) of 13-carboxy-$\gamma$-tridecalactone in the form of a pure white oil with a yield of 91%.

Step (5): Cyclization.
27 cm$^3$ (36 g) of distilled methanesulfonic acid and 4 g of phosphoric anhydride were added, in that order, to a 100 cm$^3$ flask, provided with a magnetic stirrer and thermometer, pressurised with nitrogen. The whole was kept under stirring for 1 hour at room temperature until a limpid solution was obtained. Then 1.0 g (4.13.10$^{-3}$ moles) of 13-carboxy-$\gamma$-tridecalactone were added, the temperature was raised to 60 °C and the whole was made to react for 16 hours. Then the solution was dripped into 100 cm$^3$ of water and the resultant mixture was kept under stirring for about 10 minutes. After many extractions with chloroform (4 x 30 cm$^3$), the organic phase was separated, washed with an aqueous solution of sodium bicarbonate until neutrality, dehydrated on anhydrous sodium sulphate and dried, thereby obtaining 0.97 g of a red oil. This oil was treated with diazomethane ($CH_2N_2$), in order to form the methyl ester of the desired product. The resultant oil was conveyed onto a silica gel column, using hexane-ether as eluent in a 1:1 ratio by volume. Two fractions were recovered:

1) 0.094 g (4.2.10$^{-4}$moles) of 2-(6'-carbomethoxyhexyl) cyclohex-2-ene-1-one.
2) 0.380 g (1.69.10$^{-3}$ moles) of 2(7'-carbomethoxyheptyl) cyclopent-2-ene-1-one.

The yield of the latter was 41%. The molar ratio between the cyclohexenone and the cyclopentenone was 1:4.

Examples 2-5
Following the operative conditions of Example 1, except for the esterification by means of diazomethane,

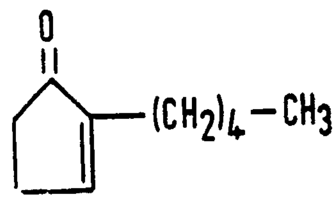

was prepared, starting from $CH_2=CH-(CH_2)_5-CH_3$, the scheme of the reactions and the yields being as follows:

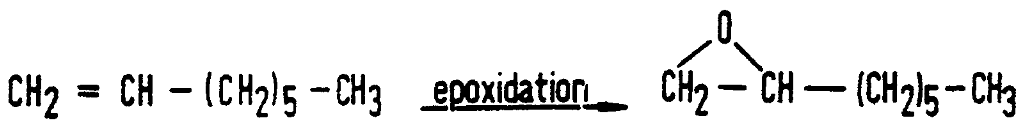


with metachloroperbenzoic acid: yield 92% with $H_2O_2$ yield 50%, selectivity 95% alkylation, saponification,

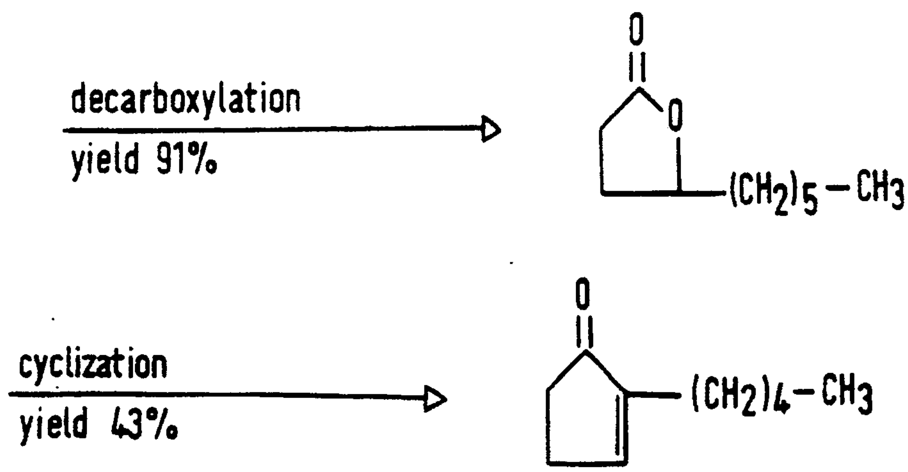


Examples 6 - 9

Following the operative conditions of Example 1, except for esterification by means of diazomethane

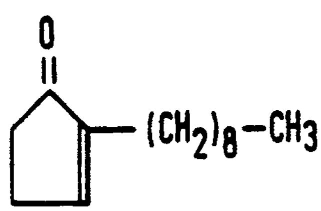

was prepared, starting from $CH_2=CH-(CH_2)_9-CH_3$, the scheme of the reactions and the yields being as follows:

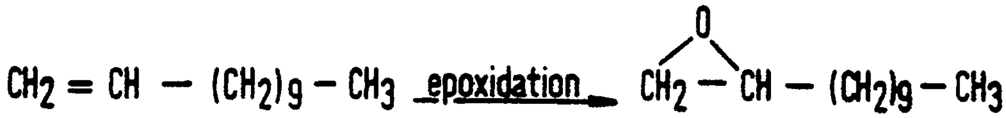

with metachloroperbenzoic acid: yield 91% with $H_2O_2$: yield 50%, selectivity 95% alkylation, saponification,

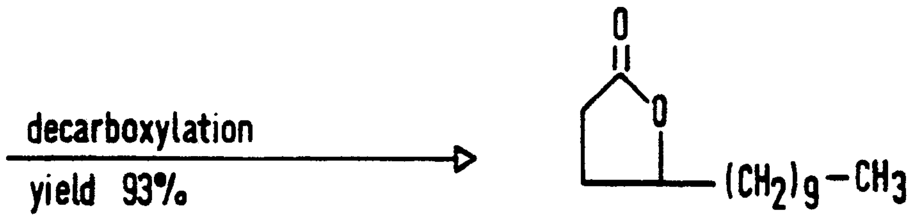


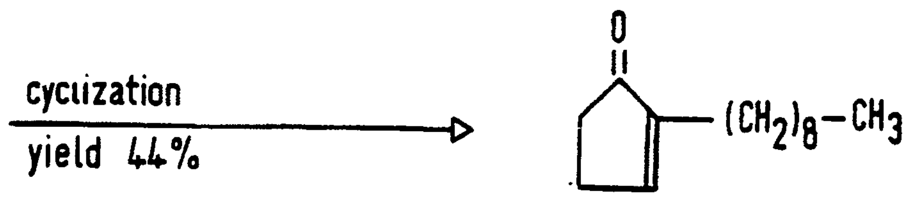


Examples 10 - 13
Following the operative conditions of Example 1.

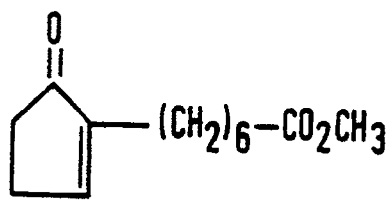

was prepared, starting from $CH_2=CH-(CH_2)_7-CO_2C_2H_5$, the scheme of the reactions and the yields being as follows:

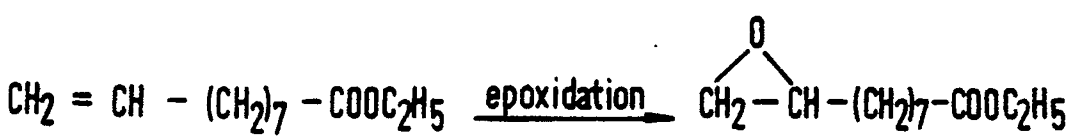


with metachloroperbenzoic acid: yield 92% with $H_2O_2$: yield 70%, selectivity 85% alkylation, saponification,

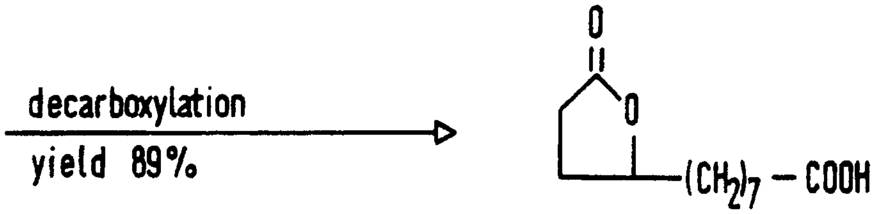


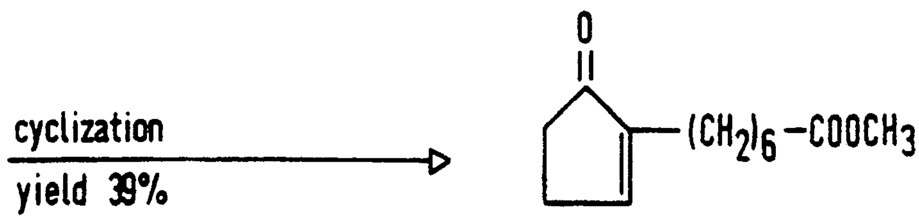


**Claims**

1. A process for the preparation of 2-alkyl-cyclopent-2-enones of formula:

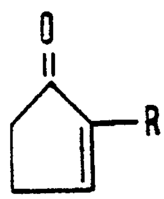

(I)

where R represents $C_nH_{2n+1}$, $(CH_2)_n$-COOR', $(CN_2)_n$-NR'$_2$, $(CH_2)_n$-OR', $(CH_2)_n$-X or $(CH_2)_n$-CN, wherein n is from 1 to 20, X represents Cl, Br, I or F, and R' is an alkyl radical containing up to 5 carbon atoms, a benzyl radical or a phenyl radical, the said benzyl or phenyl radical being optionally substituted in the aromatic ring by one or more groups inert under the reaction conditions, characterized in that the process comprises the following steps:

1) an α-olefin of formula $CH_2 = CH-CH_2-R$ (II) is oxidized to an epoxide of formula (III)

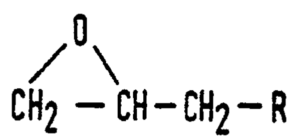

(III)

2) the epoxide (III) is reacted under reflux with an alkylating agent of formula $Na^+[CH(COOR'')_2]^-$ wherein R'' is an ethyl, isopropyl, or isobutyl radical, in the presence of a malonic ester of formula COOR''-$CH_2$-COOR'', in an alcoholic solvent of formula R''OH, the alkylating agent being used in a substantially equimolar ratio, with respect to the epoxide, and wherein said malonic ester is used in a molar ratio, with respect to the epoxide, of from 0.5 to 2, to obtain an α-carbalkoxy-γ-alkyl lactone of formula (IV)

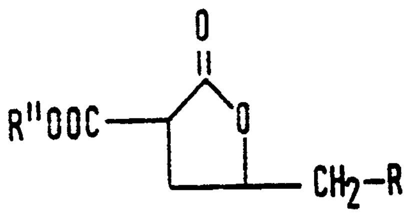

(IV)

3) the α-carbalkoxy-γ-alkyl lactone (IV) is saponified in an alkaline medium, to thereby obtain the corresponding acid;
4) the said corresponding acid is decarboxylated, at 100 - 160 °C, to thereby obtain a γ-alkyl lactone of formula (V)

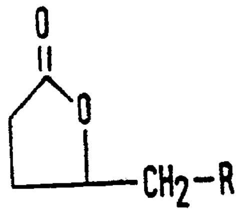

(V)

5) the γ-alkyl lactone (V) is reacted at 20 - 100 °C with a protic acid, to thereby obtain, by cyclization, a 2-alkyl-cyclopent-2-enone of formula (I).

**2.** A process as claimed in claim 1, characterized in that n is from 1 to 10.

**3.** A process as claimed in claim 1 or 2, characterized in that R is $C_nH_{2n+1}$ or $(CH_2)_n$-COOR'.

**4.** A process as claimed in any of claims 1 to 3, characterized in that the α-olefin of formula (II) is oxidized by means of m-chloroperbenzoic acid.

**5.** A process as claimed in any of claims 1 to 3, characterized in that the α-olefin of formula (II) is oxidized by means of $H_2O_2$, in the presence of a catalyst based on tungstate or phosphate ions.

**6.** A process as claimed in any of claims 1 to 5, characterized in that, in the step (2), the malonic ester is used in a molar ratio, with respect to the epoxide, of about 1.

**7.** A process as claimed in any of claims 1 to 6, characterized in that, in the step (4), the acid is decarboxylated at 120 - 140 °C.

**8.** A process as claimed in any of claims 1 to 7, characterized in that the step (5) is carried out at 50 - 60 °C.

**9.** A process as claimed in any of claims 1 to 8, characterized in that, in the step (5), the protic acid is selected from a mixture of $CH_3$-$SO_3H$ and $P_2O_5$, polyphosphoric acid and sulphuric acid.

**10.** A process as claimed in claim 9, characterized in that the protic acid is a mixture of $CH_3$-$SO_3H$ and $P_2O_5$, in a weight ratio of from 10 to 20.

**11.** A process as claimed in claim 10, characterized in that the said ratio is about 10.

**Revendications**

**1.** Un procédé pour la préparation de 2-alkyl-cyclopent-2-ènones de formule :

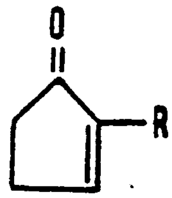

(I)

dans laquelle R représente $C_nH_{2n+1}$, $(CH_2)_n$-COOR', $(CH_2)_n$-NR'$_2$, $(CH_2)_n$-OR', $(CH_2)_n$-X ou $(CH_2)_n$-CN, où n est de 1 à 20, X represente Cl, Br, I ou F, et R' est un groupe alkyle contenant jusqu'à 5 atomes de carbone, un groupe benzyle ou un groupe phényle, le noyau aromatique des groupes benzyle ou phényle étant éventuellement substitué par un ou plusieurs groupes inertes dans les conditions réactionnelles, caractérisé en ce qu'il comprend les étapes suivantes:

1) une $\alpha$-oléfine de formule générale $CH_2 = CH\text{-}CH_2\text{-}R$ (II) est oxydée an un époxyde de formule générale (III)

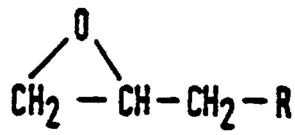

(III)

2) l'époxyde (III) est traité à reflux avec un agent d'alkylation de formule générale $Na^+[CH(COOR'')_2]^-$, dans laquelle R" est un groupe éthyle, isopropyle ou isobutyle, en présence d'un ester malonique de formule générale COOR"-$CH_2$-COOR", dans un solvant alcoolique de formule R"OH, l'agent d'alkylation étant utilisé selon un rapport sensiblement équimolaire par rapport à l'époxyde et cet ester malonique étant utilisé selon un rapport molaire de 0,5 à 2 par rapport à l'époxyde, pour obtenir une $\alpha$-carbalcoxy-$\gamma$-alkyl lactone de formule générale (IV)

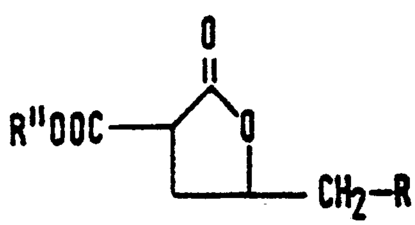

(IV)

3) l'$\alpha$-carbalcoxy-$\gamma$-alkyl lactone (IV) est saponifiée dans un milieu alcalin pour fournir l'acide correspondant;

4) l'acide correspondant est décarboxylé, à 100-160 °C, pour obtenir ainsi une $\gamma$-alkyl lactone de formule générale (V)

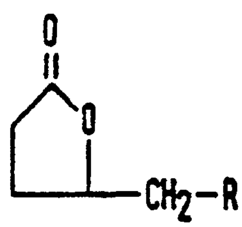

(V)

5) la $\gamma$-alkyl lactone (V) est traitée à 20-100 °C avec un acide protique pour obtenir ainsi par cyclisation, une 2-alkyl-cyclopent-2-ènone de formule générale (I).

2. Un procédé suivant la revendication 1, caractérisé en ce que n est de 1 à 10.

3. Un procédé suivant la revendication 1 ou la revendication 2, caractérisé en ce que R est un radical $C_nH_{2n+1}$ ou $(CH_2)_n$-COOR'.

4. Un procédé suivant l'une quelconque des revendications 1 à 3, caractérisé en ce que l'α-oléfine de formule (II) est oxydée au moyen d'acide m-chloroperbenzoïque.

5. Un procédé suivant l'une quelconque des revendications 1 à 3, caractérisé en ce que l'α-oléfine de formule (II) est oxydée au moyen de peroxyde d'hydrogène, en présence d'un catalyseur à base d'ions tungstate ou phosphate.

6. Un procédé suivant l'une quelconque des revendications 1 à 5, caractérisé en ce que dans l'étape (2), l'ester malonique est utilisé selon un rapport molaire d'environ 1 par rapport à l'époxyde.

7. Un procédé suivant l'une quelconque des revendications 1 à 6, caractérisé en ce que dans l'étape (4), l'acide est décarboxylé entre 120 et 140 °C.

8. Un procédé suivant l'une quelconque des revendications 1 à 7, caractérisé en ce que l'étape (5) est effectuée à 50-60 °C.

9. Un procédé suivant l'une quelconque des revendications 1 à 8, caractérisé en ce que dans l'étape (5), l'acide protique est choisi parmi un mélange de $CH_3$-$SO_3H$ et de $P_2O_5$, un acide polyphosphorique et l'acide sulfurique.

10. Un procédé suivant la revendication 9, caractérisé en ce que l'acide protique est un mélange de $CH_3$-$SO_3H$ et de $P_2O_5$, selon un rapport en poids de 10 à 20.

11. Un procédé suivant la revendication 10, caractérisé en ce que ce rapport est d'environ 10.

**Patentansprüche**

1. Verfahren zur Herstellung von 2-Alkyl-cyclopent-2-enonen der Formel

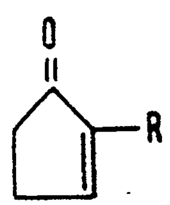

(I)

worin R $C_nH_{2n+1}$, $(CH_2)_n$-COOR', $(CH_2)_n$-NR'$_2$, $(CH_2)_n$-OR', $(CH_2)_n$-X oder $(CH_2)_n$-CN darstellt, worin n 1 bis 20 ist, X Cl, Br, I oder F repräsentiert und R' einen Alkylrest mit bis zu 5 Kohlenstoffatomen, einen Benzylrest oder einen Phenylrest bedeutet, wobei der Benzyl- oder Phenylrest gegebenenfalls am aromatischen Ring durch eine oder mehrere Gruppen, welche unter den Reaktionsbedingungen inert sind, substituiert ist, dadurch gekennzeichnet, daß das Verfahren die folgenden Schritte umfaßt:

1) ein α-Olefin der Formel $CH_2=CH-CH_2R$ (II) wird zu einem Epoxid der Formel (III) oxidiert;

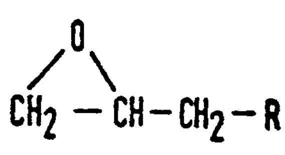

(III)

2) das Epoxid (III) wird unter Rückfluß mit einem Alkylierungsmittel der Formel $Na^+[CH(COOR'')_2]^-$,

worin R" einen Ethyl-, Isopropyl- oder Isobutylrest darstellt, in Gegenwart eines Malonesters der Formel COOR"-$CH_2$-COOR" in einem alkoholischen Lösungmittel der Formel R"OH umgesetzt, wobei das Alkylierungsmittel, bezogen auf das Epoxid, in einem im wesentlichen äquimolaren Verhältnis eingesetzt wird und worin der Malonester in einem Molverhältnis, bezogen auf das Epoxid, von 0,5 bis 2 eingesetzt wird, um ein α-Carbalkoxy-γ-alkyllacton der Formel (IV) zu erhalten

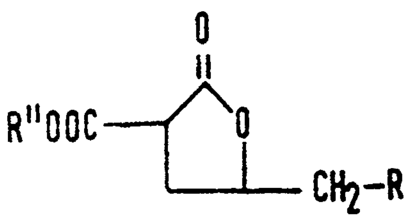

(IV)

3) das α-Carbalkoxy-γ-alkyllacton (IV) wird in einem alkalischen Medium verseift, um dadurch die korrespondierende Säure zu erhalten;
4) die korrespondierende Säure wird bei 100 bis 160 °C decarboxyliert, um dadurch ein γ-Alkyllacton der Formel (V) zu erhalten

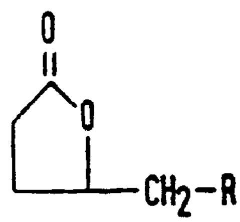

(V)

5) das γ-Alkyllacton (V) wird bei 20 bis 100 °C mit einer protischen saure umgesetzt, um dadurch durch Cyclisierung ein 2-Alkylcyclopent-2-enon der Formel (I) zu erhalten.

**2.** Verfahren nach Anspruch 1, dadurch gekennzeichnet, daß n 1 bis 10 darstellt.

**3.** Verfahren nach Anspruch 1 oder 2, dadurch gekennzeichnet, daß R $C_nH_{2n+1}$ oder $(CH_2)_n$-COOR' darstellt.

**4.** Verfahren nach irgendeinem der Ansprüche 1 bis 3, dadurch gekennzeichnet, daß das α-Olefin der Formel (II) mit m-Chlorperbenzoesäure oxidiert wird.

**5.** Verfahren nach irgendeinem der Ansprüche 1 bis 3, dadurch gekennzeichnet, daß das α-Olefin der Formel (II) mit $H_2O_2$ oxidiert wird, in Gegenwart eines Katalysators auf der Basis von Wolframat- oder Phosphationen.

**6.** Verfahren nach irgendeinem der Ansprüche 1 bis 5, dadurch gekennzeichnet, daß im Schritt (2) der Malonester in einem Molverhältnis, bezogen auf das Epoxid, von ungefähr 1 eingesetzt wird.

**7.** Verfahren nach irgendeinem der Ansprüche 1 bis 6, dadurch gekennzeichnet, daß im Schritt (4) die Säure bei 120 bis 140 °C decarboxyliert wird.

**8.** Verfahren nach irgendeinem der Ansprüche 1 bis 7, dadurch gekennzeichnet, daß der Schritt (5) bei 50 bis 60 °C durchgeführt wird.

**9.** Verfahren nach irgendeinem der Ansprüche 1 bis 8, dadurch gekennzeichnet, daß im Schritt (5) die protische Säure aus einer Mischung von $CH_3$-$SO_3H$ und $P_2O_5$, Polyphosphorsäure und Schwefelsäure ausgewählt ist.

**10.** Verfahren nach Anspruch 9, dadurch gekennzeichnet, daß die protische Säure eine Mischung von $CH_3$-

$SO_3H$ und $P_2O_5$ in einem Gewichtsverhältnis von 10 bis 20 darstellt.

**11.** Verfahren nach Anspruch 10, dadurch gekennzeichnet, daß das Verhältnis ungefähr 10 beträgt.